# EUROPEAN PATENT APPLICATION

(11) **EP 4 850 694 A1**
(43) Date of publication of application: **09.12.2026**
(21) Application number: 25747882.6
(22) Date of filing: 24.01.2025
(51) Int. Cl.: A61K 45/06, A61K 31/536, A61K 31/519, A61K 39/395, A61P 35/00

(54) **PHARMACEUTICAL COMPOSITION CONTAINING PRMT5 INHIBITOR AND PD-1/PD-L1 INHIBITOR**

(30) Priority: 02.02.2024 CN 202410153995; 22.02.2024 CN 202410200806; 04.03.2024 CN 202410245467; 25.03.2024 CN 202410346358
(71) Applicant: Apeiron Therapeutics (Hong Kong) Ltd., Hong Kong 999077 (HK)
(72) Inventor: YAO, Bing, Shanghai 201210 (CN); MAO, Weifeng, Shanghai 201210 (CN); ASWAD, Fred Jullien, Shanghai 201210 (CN); LI, Mingxi, Shanghai 201210 (CN); GU, Xiaohui, Shanghai 201210 (CN); JOSEPH, James David, Shanghai 201210 (CN)
(74) Representative: Bayramoglu et al.
(86) International application number: PCT/CN2025/074930
(87) International publication number: WO 2025/162314

(57) **Abstract**

A pharmaceutical composition is provided, including a pharmaceutical composition or a combined pharmaceutical composition of a protein arginine methyltransferase 5 (PRMT5) inhibitor and a PD-1/PD-L1 inhibitor. The pharmaceutical composition can be used to treat various cancers, including solid tumors.

## Description

### TECHNICAL FIELD

The present disclosure belongs to the field of medicine, and specifically relates to a pharmaceutical composition comprising a PRMT5 inhibitor and a PD-1/PD-L1 inhibitor.

### BACKGROUND ART

Cancer is among the leading causes of death worldwide. Popular treatments, such as chemotherapy and immunotherapy, are limited in that their cytotoxic effects are not restricted to cancer cells and can also cause adverse side effects in normal tissues.

PRMT5 is a type II arginine methyltransferase that regulates important cellular functions, including cell cycle progression, apoptosis, and the DNA damage response, by symmetrically dimethylating proteins involved in transcription and signal transduction. However, data from genome-wide genetic perturbation screens using shRNA revealed a selective requirement for PRMT5 activity in MTAP-deleted cancer cell lines (Kruykov et al., 2016; Marjon et al., 2016, and Markarov et al., 2016). The accumulation of MTA caused by MTAP deletion in these cell lines partially inhibits PRMT5, making these cells selectively sensitive to additional PRMT5 inhibition.

Certain PRMT5 inhibitors have been developed, but they do not show selectivity for MTAP-deleted cancer cell lines. This lack of selectivity could be explained by the inhibitors' mechanism of action, as they are either SAM-uncompetitive or SAM-competitive inhibitors and therefore independent of MTAP (Kruykov et al., 2016; Marjon et al., 2016, and Markarov et al., 2016).

By using inhibitors that bind PRMT5 non-competitively or cooperatively with MTA, selectivity for MTAP-deleted/MTA-accumulating cells can be improved. PRMT5 inhibitors that bind non-competitively or cooperatively with MTA will exhibit increased binding to PRMT5 in the presence of MTA compared to the binding of the same inhibitor in the absence of MTA. Consequently, such inhibitors will bind with significantly greater potency in the presence of high concentrations of MTA and, therefore, lead to preferential inhibition of PRMT5 in MTA-accumulating cells relative to normal cells.

Although standard treatments for many different types of cancer have been improved greatly over the years, current standard treatments still cannot fully meet the needs of improving cancer treatment. Recently, the clinical application of immuno-oncology drugs targeting programmed cell death protein 1 (PD-1) and its ligand PD-L1 has been improved in the treatment of many types of cancer. Although these checkpoint inhibitors have improved clinical responses for some cancers, durable clinical responses only occur in about 10-45% of patients. In addition, many tumors either become resistant to treatment or become difficult to treat. Therefore, new treatments are currently needed, including combination therapies for cancers.

The chromosomal 9p21 locus containing the tumor suppressor CDKN2A/B and methylthioadenosine phosphorylase (MTAP) is one of the most common gene deletions in a cancer. The 9p21 deletion is associated with a decrease in tumor-infiltrating lymphocytes (TIL) and resistance to immune checkpoint inhibitor (ICI) treatment. Previously these were thought to be caused by CDKN2A/B deletion, Donjeta Gjuka et al. found that it was the MTAP deletion that led to poor ICI treatment and reduced TIL density. MTAP deletion can lead to the accumulation of intracellular and extracellular methylthioadenosine (MTA) and severely impair T cell function by inhibiting protein arginine methyltransferase 5 (PRMT5) and the adenosine receptor agonism (Cancer Cell, 2023, DOI: 10.1016/j.ccell.2023.09.005). Administering MTA-cooperative PRMT5 inhibitors has the potential to reverse this immunosuppressive effect, increase TIL, and inhibit tumor growth, potentially providing significant benefits for patients with ineffective/low 9p21/MTAP.

### SUMMARY OF INVENTION

In order to address the technical problem, the present disclosure provides a pharmaceutical composition comprising a PRMT5 inhibitor of Formula (I) as a first active ingredient and a chemotherapeutic agent as a second active ingredient:

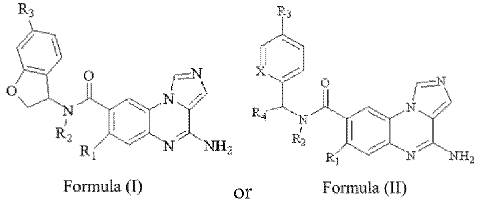

Wherein, in Formula (I) or Formula (II),
R₁ is H, halogen, C₁-C₆ alkyl, C₁-C₆ haloalkyl, and CN;
R₂ is H, C₁-C₆ alkyl, C₁-C₆ haloalkyl, C₁-C₆ deuteroalkyl, and C₃-C₆ cycloalkyl;
R₃ is H, halogen, C₁-C₆ alkyl, C₁-C₆ haloalkyl, C₁-C₆ haloalkoxy, or SF₅;
in Formula II, R₄ is hydrogen or C₁-C₆ alkyl; X is CR₅ or N;
Wherein, R₅ is hydrogen, halogen, C₁-C₆ alkyl, C₁-C₆ haloalkyl, hydroxyl, -NH₂, or CN.

In a preferred embodiment of the present disclosure, wherein, in Formula (I) or Formula (II):
R₁ is selected from hydrogen, halogen, C₁-C₆ alkyl, and halo(C₁-C₆ alkyl);
R₂ is selected from hydrogen, C₁-C₆ alkyl, C₁-C₆ haloalkyl, C₁-C₆ deuteroalkyl, and C₃-C₆ cycloalkyl;
R₃ represents hydrogen, halogen, C₁-C₆ haloalkyl, C₁-C₆ haloalkoxy, or SF₅;
R₄ represents hydrogen or methyl;
and in Formula II, X is CH or N.

In one preferred embodiment of the present disclosure, wherein, R₁ is hydrogen or fluorine.

In one preferred embodiment of the present disclosure, wherein, R₂ is cyclopropyl, methyl or deuterated methyl.

In one preferred embodiment of the present disclosure, wherein, R₃ is CF₃.

In one preferred embodiment of the present disclosure, wherein, R₄ is hydrogen or methyl.

In one preferred embodiment of the present disclosure, wherein, X is N.

In one preferred embodiment of the present disclosure, wherein, the PRMT5 inhibitor as the first active ingredient is selected from the group consisting of the following compounds and combinations thereof:

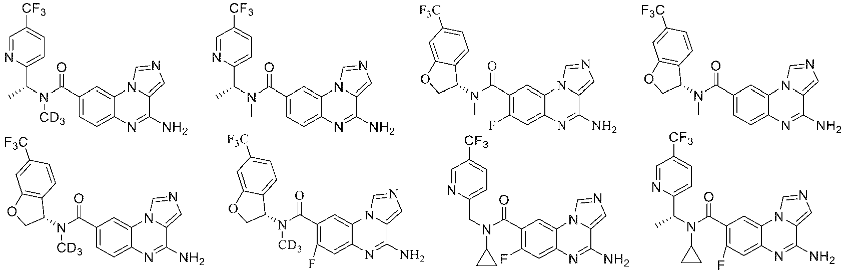

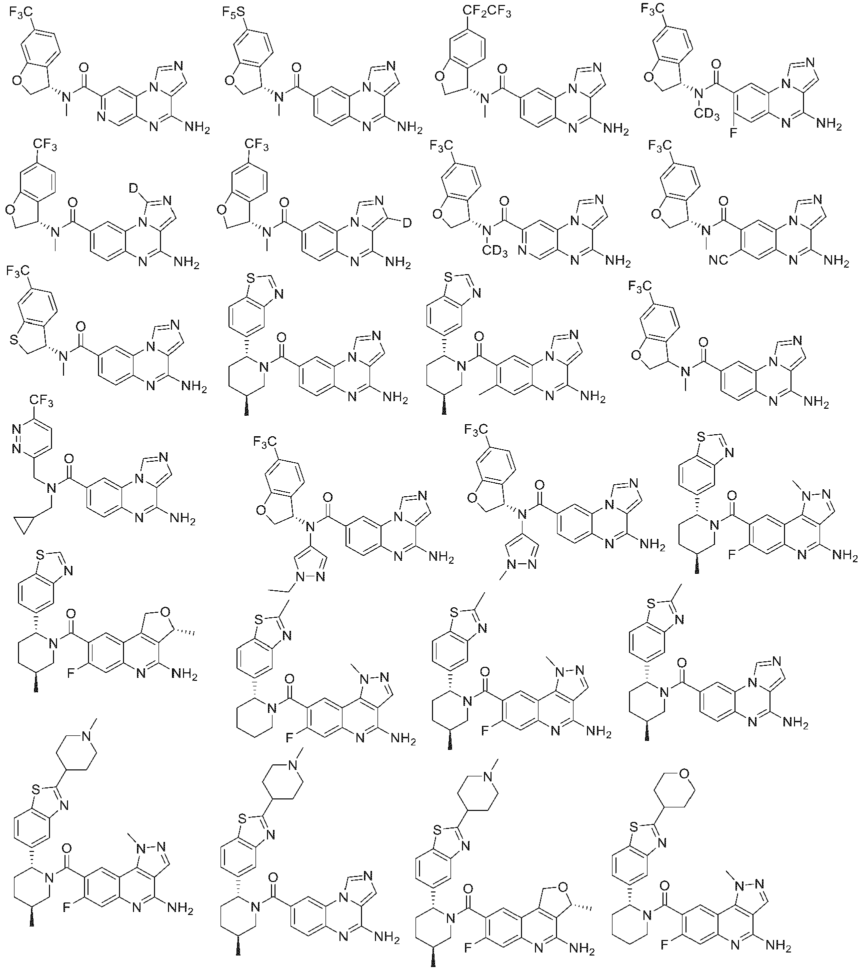

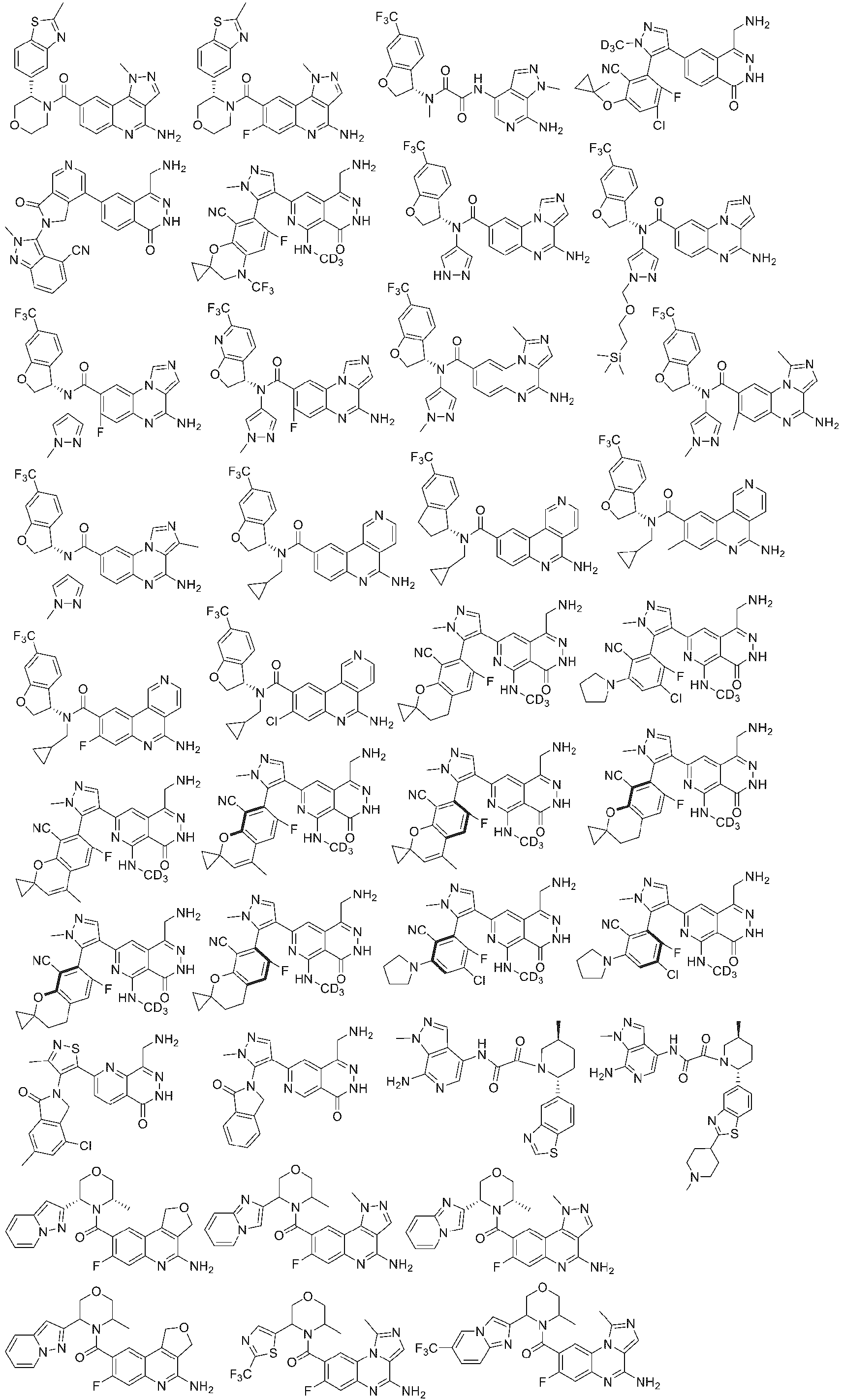

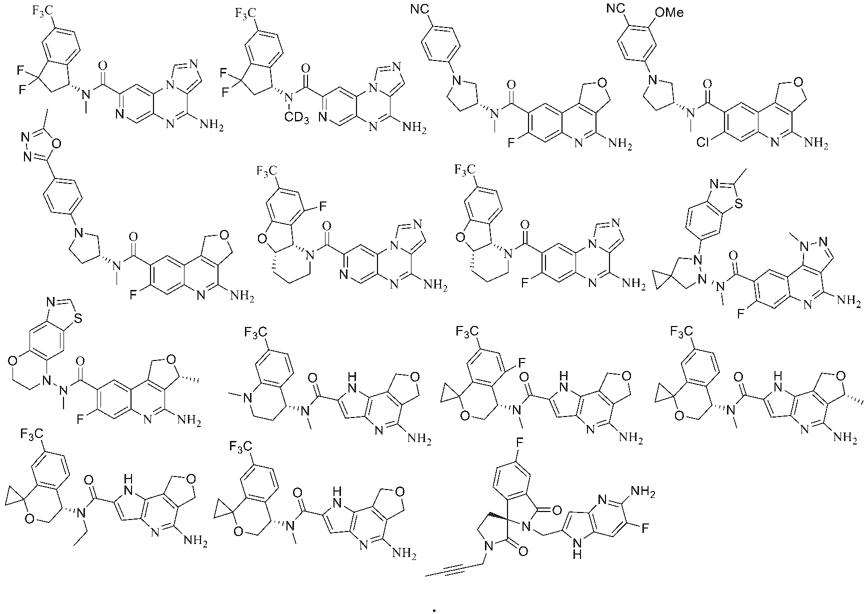

In one preferred embodiment of the present disclosure, wherein, the PD-1/PD-L1 inhibitor as the second active ingredient is a small molecule compound, nucleic acid, peptide, protein, antibody, peptide antibody, double antibody, mini antibody, single chain fragment variable (ScFv) or a fragment or variant or any combination thereof that can be used to interfere with the interaction between PD-1 and PD-L1 to stimulate antitumor immune response, but is not limited by any binding theory.

In one preferred embodiment of the present disclosure, wherein, the PD-1/PD-L1 inhibitor as the second active ingredient is an antibody selected from anti-PD-1 antibody (or "PD-1 antibody" for short) and anti-PD-L1 antibody (or "PD-L1 antibody" for short).

In one preferred embodiment of the present disclosure, wherein, the anti-PD-1/PD-L1 inhibitor is a monoclonal PD-1 antibody selected from nivolumab (OPDIVO^{®}), Pembrolizumab (Keytruda^{®}), Pidilizumab, Tislelizumab (Bai Ze'an^{®}), Sintilimab (Tyvyt^{®}), Penpulimab (Anniko^{®}), Dostarlimab (Jemperli^{®}), Cemiplimab (Libtayo^{®}), Toripalimab (Loqtorzi^{®}), Tislelizumab (Tevimbra^{®}), Retifanlimab (Zynyz^{®}), Spartalizumab (PDR001), Camrelizumab (SHR-1210), MEDI0680 or RMP1-14 (rat IgG) or any combination thereof.

In one preferred embodiment of the present disclosure, wherein, the PD-1/PD-L1 inhibitor is a monoclonal PD-L1 antibody selected from Atezolizumab (TECENTRIQ^{®}), Envafolimab (Envida^{®}, KN035), Avelumab (BAVENCIO^{®}), Durvalumab (Imfinzi^{®}), BMS936559 or any combination thereof.

In one preferred embodiment of the present disclosure, wherein, the anti-PD-1/PD-L1 inhibitor is a small molecule compound selected from ASC61, BPI-371153, AN4005, INCB086550, MAX-10181 or Emdiphene (IMMH-010) or any combination thereof.

In another aspect, the present disclosure provides a method of treating cancer or a tumor, comprising administering the pharmaceutical composition of the present disclosure to a subject in need thereof..

In one preferred embodiment of the present disclosure, wherein, the tumor or cancer is selected from: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, bile duct cancer, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, epidermal squamous cell carcinoma, nasopharyngeal carcinoma, neuroblastoma, endometrial cancer, head and neck cancer, and cervical cancer.

As will be understood by those of ordinary skill in the art, in any embodiment disclosed herein, any feasible combination of a compound of formula (I) or a pharmaceutically acceptable salt, solvate or prodrug thereof and a compound of formula (II) or a pharmaceutically acceptable salt, solvate or prodrug thereof is included in the present disclosure, as long as such combination is capable of producing some synergistic effect in the treatment of a subject in need of such treatment.

When any compound is used in the present disclosure, it includes any pharmaceutically acceptable form thereof, including but not limited to isomers, tautomers, salts, solvates, polymorphs, prodrugs, and so on. It should be understood that the term "compound" includes any and all such forms, whether or not explicitly stated, although sometimes only certain terms are explicitly stated, such as "salt" and "prodrug".

Unless expressly defined otherwise, all terms used herein have the ordinary meaning as would be interpreted or understood by one of ordinary skill in the art.

The terms "a", "an" or "the" used herein refer to both the singular and the plural forms. Generally, when a singular or plural form of a noun is used, it refers to both the singular and the plural forms of the noun.

When the term "about" is applied to a parameter, it means that the parameter can vary within +10%, preferably within ±5%, including any number from the lower limit to the upper limit. When the term "about" is applied to a range, it applies to both the lower and upper limits of the range. As will be understood by those skilled in the art, when a parameter is not critical, a number is generally given for illustrative purposes only and is not limited.

"Alkoxy" refers to a group -OR, wherein, R is alkyl as defined herein. Representative examples include methoxy, ethoxy, propoxy, isopropoxy, sec-butoxy, tert-butoxy, and the like.

"Alkyl" refers to a group derived from a straight or branched chain saturated hydrocarbon by removing a hydrogen from one of the saturated carbons. Alkyl groups preferably contain 1 to 8 carbon atoms, sometimes preferably 1 to 6 carbon atoms, and sometimes even more preferably 1 to 4 carbon atoms. Representative examples of alkyl groups include, but are not limited to, methyl, ethyl, n-propyl, isopropyl, n-butyl, sec-butyl, tert-butyl, pentyl, hexyl, and the like. "Lower alkyl," "lower alkoxy," or "lower haloalkyl" refers to an alkyl or alkyl moiety having one to four, sometimes preferably one to three or one to two carbon atoms.

As used herein, the term "cyano" refers to -CN.

The term "cycloalkyl" as used herein refers to a group derived from a monocyclic saturated carbocyclic ring by removing a hydrogen atom from the saturated carbocyclic ring, preferably having 3 to 8, more preferably 3 to 6 carbon atoms. Representative examples of cycloalkyl include, but are not limited to, cyclopropyl, cyclopentyl, and cyclohexyl.

As used herein, the terms "halo" and "halogen" refer to F, Cl, Br, or I.

The term "haloalkyl" as used herein refers to an alkyl group substituted with at least one halogen atom. A haloalkyl group can be an alkyl group in which all hydrogen atoms are substituted with halogens. Representative examples of haloalkyl groups include, but are not limited to, trifluoromethyl, fluoromethyl, difluoromethyl, bromomethyl, 1-chloroethyl, perchloroethyl, 2-fluoroethyl, and so on.

The term "heterocyclyl" as used herein refers to a 3 to 10-membered monocyclic or bicyclic non-aromatic group containing one or more, preferably 1 to 3, heteroatoms independently selected from nitrogen (N), oxygen and sulfur (S, S(O) or S(O)₂) in the non-aromatic ring. The heterocyclyl of the present disclosure can be connected to the parent molecular moiety through a carbon atom or a nitrogen atom in the group. The heterocyclyl group can be saturated or unsaturated, for example, containing one or more double bonds in the ring. Unless otherwise stated, the valence of the group can be located on any atom of any ring within the group where the valence rules permit. Examples may include, but are not limited to, azetidinyl, pyrrolidinyl, 2-oxopyrrolidinyl, 2,5-dihydro-1H-pyrrolyl, piperidinyl, 4-piperidinyl, morpholinyl, piperazinyl, 2-oxopiperazinyl, tetrahydropyranyl, tetrahydrofuranyl, 2-oxopiperidinyl, thiomorpholinyl, pyrazolidinyl, imidazolinyl, imidazolidinyl, and so on.

When any group, such as "cycloalkyl" or "heterocyclyl" is referred to as "substituted or unsubstituted" or "optionally substituted", unless otherwise specified, it means that the group is substituted or not substituted by 1 to 5, sometimes preferably 1 to 3 or 1 to 2 substituents independently selected from halogen, C₁-C₆ alkyl, C₁-C₆ alkoxy, C₁-C₆ haloalkyl, and cyano.

The term "solvate" as used herein refers to a physical association of a compound of the invention with one or more, preferably one to three, solvent molecules (whether organic or inorganic). This physical association includes hydrogen bonding. In some cases, the solvate is capable of separation, for example when one or more, preferably one to three, solvent molecules are incorporated into the crystal lattice of a crystalline solid. Exemplary solvates include, but are not limited to, hydrates, ethanolates, methanolates, and isopropanolates. Solvation methods are generally known in the art.

"Prodrugs" refer to compounds that can be converted in vivo to produce the active parent compound under physiological conditions, such as by hydrolysis in the blood. Common examples include, but are not limited to, ester and amide forms of compounds having an active form with a carboxylic acid moiety. Amides and esters of the compounds of the present invention can be prepared according to conventional methods. In particular, in the present invention, prodrugs can also be formed by acylation of the amino group or nitrogen atom in the heterocyclyl ring structure, where the acyl group can hydrolyze in vivo. Such acyl groups include but are not limited to C₁-C₆ acyl groups, preferably C₁-C₄ acyl groups, more preferably C₁-C₂ (formyl or acetyl) groups, or benzoyl groups.

As used herein, the term "subject" refers to a human or other mammal, such as a monkey, dog, cat, or horse. The term is intended to encompass and is sometimes interchangeable with "patient".

As used herein, the terms "administering" or "drug administration" refer to providing a compound or pharmaceutical composition to a subject having or at risk for a disease or condition to be treated or prevented.

Any route of administration is suitable for the present disclosure. In one embodiment, the compounds of the present disclosure can be administered to a subject in a solid dosage form such as a tablet, capsule, or the like. In one embodiment, the compounds of the present disclosure can be administered to a subject by intravenous injection. In another embodiment, the compounds of the present disclosure can be administered to a subject by any other suitable systemic delivery method, such as oral, parenteral, intranasal, sublingual, rectal or transdermal administration.

As used herein, the term "therapeutically effective amount" refers to that amount of a compound or composition that will elicit the desired or intended biological or medical response in a subject that is being sought by a physician, veterinarian, or researcher. The therapeutically effective amount of the compound and the specific pharmaceutically acceptable carrier will vary depending on, for example, the age, weight, sex of the subject, the mode of administration, and the disease or condition being treated.

The term "pharmaceutically acceptable" when used before a compound, salt, prodrug, composition, or carrier means that such compound, salt, prodrug, composition, or carrier is suitable for administration to a subject for treatment without causing intolerable side effects to the subject considering the desired treatment.

As used herein, the term "pharmaceutically acceptable carrier" refers to a substance that is compatible with the compounds used in the present invention and can be used to administer the compounds in the methods of the present invention, and is preferably non-toxic, or inert and pharmaceutically acceptable. Pharmaceutically acceptable carriers can be solid, liquid or gaseous substances, including any and all dry powders, solvents, dispersion media, coatings, antibacterial and antifungal agents, isotonic agents, absorption delaying agents, and the like. Examples of such carriers include oils such as corn oil, buffers such as phosphate buffered saline (PBS), saline, polyethylene glycol, glycerol, polypropylene glycol, dimethyl sulfoxide, amides such as dimethylacetamide, proteins such as albumin, detergents such as Tween 80, monosaccharides and oligosaccharides such as glucose, lactose, cyclodextrins, starch, and the like.

As described herein, some embodiments of the compounds of the present disclosure can contain basic functional groups, such as amino or alkylamino, and therefore can form pharmaceutically acceptable salts with pharmaceutically acceptable acids. In this respect, the term "pharmaceutically acceptable salts" refers to relatively nontoxic inorganic and organic acid addition salts of the compounds of the present disclosure. These salts can be prepared on site during the administration of carriers or dosage form production processes, or by reacting the purified compounds of the present invention in free alkali form with suitable organic or inorganic acids alone, and separating the salts so formed in subsequent purification processes to prepare. Representative salts include hydrobromide, hydrochloride, sulfate, bisulfate, phosphate, nitrate, acetate, valerate, oleate, palmitate, stearate, laurate, benzoate, lactate, phosphate, toluenesulfonate, citrate, maleate, fumarate, and succinate.

The formulations used in the present disclosure may also contain stabilizers, preservatives, buffers, antioxidants or other additives known to those skilled in the art. The use of such media and agents for pharmaceutically active substances is well known in the art.

The terms "synergistic" and the like as used herein refer to an effect caused by a combination of two or more agents that is greater than the cumulative effect of the two or more agents used alone. This synergistic effect of combination therapy includes higher efficacy, lower side effects, or both. In some embodiments, the synergistic effect includes a significant reduction in the side effects of the two therapeutic inhibitors due to a reduction in the dosage of the two therapeutic inhibitors, while the overall therapeutic efficacy remains at approximately the same or improved levels. In some embodiments, the synergistic effect includes a significant improvement in the efficacy of inhibiting cancer cell proliferation, while the side effects caused by the two drugs remain at approximately the same or lower levels. The synergistic effect allows the use of a lower dose of a single drug to effectively treat the disease. In general, the synergistic combination of two or more drugs can lead to improvements in disease treatment compared to monotherapy.

Combination therapy can allow the use of lower doses of a first therapeutic agent, such as a PRMT5 inhibitor, or a second therapeutic agent, such as a PD-1/PD-L1 inhibitor, or lower doses of both therapeutic agents than would normally be required when either agent is used alone. The present disclosure encompasses any and all such "synergistic" effects.

The pharmaceutical composition may contain a PRMT5 inhibitor and a PD-1/PD-L1 inhibitor for use in the methods of the present disclosure in an amount ranging from 0.01% to 99% by weight of the total composition, preferably from 0.1% to 80% by weight of the total composition, and more preferably from 0.1% to 50% by weight of the total composition. The weight ratio between the PRMT5 inhibitor and the PD-1/PD-L1 inhibitor may be in the range of 1:20 to 20:1, sometimes preferably from 1:15 to 15:1, and sometimes more preferably from 1:10 to 10:1.

For systemic administration, the daily dosage range for adult human treatment of a PRMT5 inhibitor is about 0.01 to about 150 mg/kg, preferably about 0.05 to about 100 mg/kg, and sometimes more preferably about 0.1 to about 50 mg/kg.

The present disclosure provides pharmaceutical compositions that can be used to treat and/or prevent various cancers that may include or exclude the following cancers: glioblastoma multiforme, brain cancer, prostate cancer, pancreatic cancer, mantle cell lymphoma, non-Hodgkin lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, bile duct cancer, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, epidermal squamous cell carcinoma, nasopharyngeal carcinoma, neuroblastoma, endometrial cancer, head and neck cancer, and cervical cancer.

In a preferred embodiment of the present disclosure, wherein, the cancer is metastatic cancer.

In a preferred embodiment of the present disclosure, wherein, the metastatic cancer is brain metastatic cancer.

### DESCRIPTION OF DRAWINGS

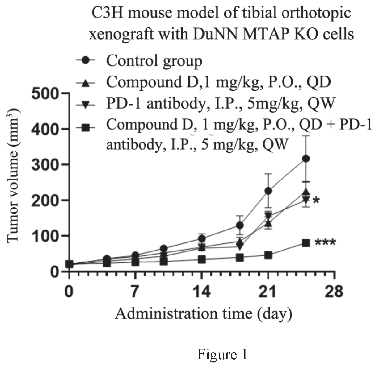

**Figure 1** **In vivo pharmacodynamic study of compound D in C3H mouse model of tibial orthotopic xenograft with DuNN MTAP KO cells**

### SPECIFIC EXAMPLES

The information of PD-1 antibody used in this test is: 29F.1A12^{™} monoclonal antibody (inVivoMab anti-mouse PD-1), purchased from BioXCell, item number: #BE0273.

The chemical name of compound A used in this test is (S)-4-amino-N-methyl-N-(6-(trifluoromethyl)-2,3-dihydrobenzofuran-3-yl)imidazo[1,5-a]quinoxaline-8-carboxamide, which has the following chemical structure:

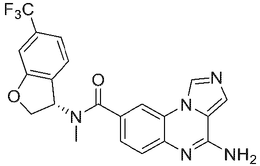

The chemical name of compound B used in this test is: 4-amino-N-cyclopropyl-7-fluoro-N-(5-(trifluoromethyl)pyridin-2-yl)methyl)imidazo[1,5-a]quinoxaline-8-carboxamide, which has the following chemical structure:

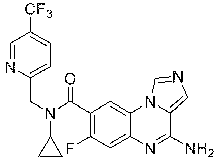

The chemical name of compound C used in this test is: (R)-4-amino-N-cyclopropyl-7-fluoro-*N*-(1-(5-trifluoromethyl)pyridin-2-yl)ethyl)imidazo[1,5-a]quinoxaline-8-carboxamide, which has the following chemical structure:

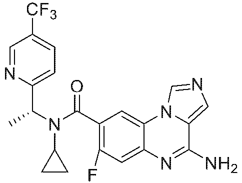

The chemical name of compound D used in this test is: (*S*)-4-amino-7-fluoro-*N*-methyl-*N*-(6-trifluoromethyl)-2,3-dihydrobenzofuran-3-yl)imidazo[1,5-a]quinoxaline-8-carboxamide, which has the following chemical structure:

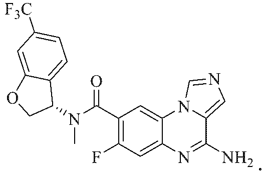

The chemical name of compound E used in this test is: (S)-4-amino-N-methyl-N-(6-(trifluoromethyl)-2,3-dihydrobenzofuran-3-yl)imidazo[1,5-a]pyrido[3,4-e]pyrazine-8-carboxamide, which has the following chemical structure:

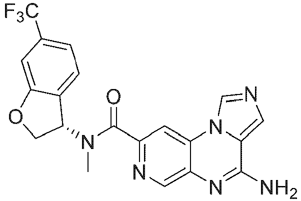

The chemical name of compound F used in this test is: (S)-4-amino-N-(methyl-d₃)-N-(6-(trifluoromethyl)-2,3-dihydrobenzofuran-3-yl)imidazo[1,5-a]quinoxaline-8-carboxamide, which has the following chemical structure:

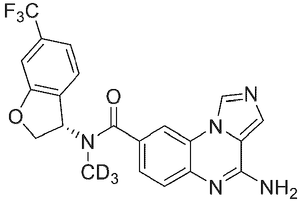

The chemical name of compound G used in this test is: (S)-4-amino-N-methyl-N-(6-(pentafluoro-λ⁶-sulfane)-2,3-dihydrobenzofuran-3-yl)imidazo[1,5-a]quinoxaline-8-carboxamide, which has the following chemical structure:

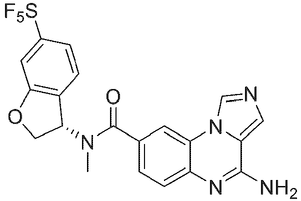

The chemical name of compound H used in this test is: (S)-4-amino-N-methyl-N-(6-(perfluoroethyl)-2,3-dihydrobenzofuran-3-yl)imidazo[1,5-a]quinoxaline-8-carboxamide, which has the following chemical structure:

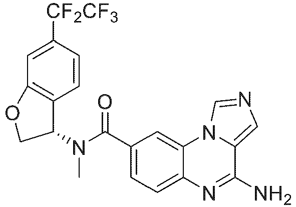

The chemical name of compound I used in this test is: (S)-4-amino-7-fluoro-N-(methyl-d₃)-N-(6-(trifluoromethyl)-2,3-dihydrobenzofuran-3-yl)imidazo[1,5-a]quinoxaline-8-carboxamide, which has the following chemical structure:

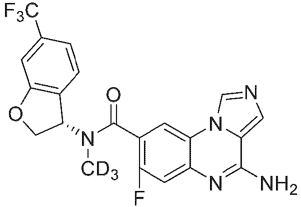

The chemical name of compound J used in this test is: (S)-4-amino-N-methyl-N-(6-(trifluoromethyl)-2,3-dihydrobenzofuran-3-yl)imidazo[1,5-a]quinoxaline-8-carboxamide-1-d, which has the following chemical structure:

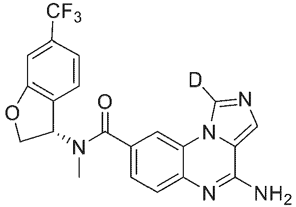

The chemical name of compound K used in this test is (S)-4-amino-N-methyl-N-(6-(trifluoromethyl)-2,3-dihydrobenzofuran-3-yl)imidazo[1,5-a]quinoxaline-8-carboxamide-3-d, which has the following chemical structure:

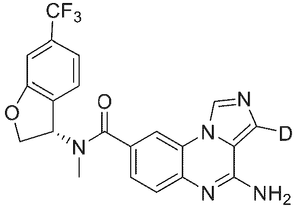

The chemical name of compound L used in this test is: (S)-4-amino-N-(methyl-d₃)-N-(6-(trifluoromethyl)-2,3-dihydrobenzofuran-3-yl)imidazo[1,5-a]pyrido[3,4-e]pyrazine-8-carboxamide, which has the following chemical structure:

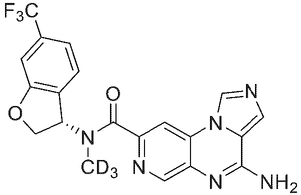

The chemical name of the compound M used in this test is: (S)-4-amino-7-cyano-N-methyl-N-(6-(trifluoromethyl)-2,3-dihydrobenzofuran-3-yl)imidazo[1,5-a]quinoxaline-8-carboxamide, which has the following chemical structure:

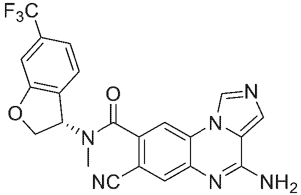

The chemical name of the compound N used in this test is: (R)-4-amino-N-(methyl-d₃)-N-(1-(5-(trifluoromethyl)pyridin-2-yl)ethyl)imidazo[1,5-a]quinoxaline-8-carboxamide, which has the following chemical structure:

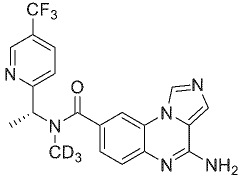

The chemical name of compound O used in this test is: (R)-4-amino-N-methyl-N-(1-(5-(trifluoromethyl)pyridin-2-yl)ethyl)imidazo[1,5-a]quinoxaline-8-carboxamide, which has the following chemical structure:

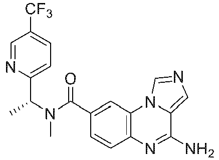

The preparation of Compounds A, D, E, F, G, H, I, J, K, L, and M is described in international patent application PCT/CN2023/120923, and the preparation of Compounds B, C, N, and O is described in international patent application PCT/CN2023/111604, the entirety of each of which is incorporated herein by reference

### Example 1: In vivo pharmacodynamic study of compound D in C3H mouse model of tibial orthotopic xenograft with DuNN MTAP KO cells

Cell culture: Human osteosarcoma DuNN MTAP KO cells (provided by the Experimental Animal Center of Shanghai First People's Hospital) were cultured in vitro monolayer under the conditions of adding 10% fetal bovine serum and 1% Antibiotic-Antimycotic to DMEM medium, and culturing in a 5% CO₂ incubator at 37°C. Pancreatin-EDTA was used twice a week for routine digestion and passage. When the cell saturation is 80%-90% and the number of cells reached the requirement, the cells were collected, counted, and inoculated.

Animals: C3H normal mice, female, 4-6 weeks old, weighing 18-22 g. A total of 30 animals were required (24 enrolled, surplus).

Tumor inoculation: DUNN cells were digested with 0.25%pancreatin-EDTA solution to make them into a single cell suspension, washed with PBS buffer and the cell density was adjusted to 1×10⁷/ml for further use. On the day of the experiment, mice were intraperitoneally injected with 0.5% sodium pentobarbital solution at a dose of 40 mg/kg to anesthetize them. A bone hole with a diameter of about 1 mm was drilled in the upper end of the right hindlimb tibia with a 1 ml syringe to reach the marrow cavity. Be careful not to penetrate the contralateral cortical bone and cause fractures or bleeding. A 1ml syringe and a 25G needle were used to draw the DUNN cell suspension and it was slowly injected into the tibial bone marrow cavity of the experimental group mice at a dose of 30µl. Be careful not to cause cell reflux or leakage. After injection, the surgical field was disinfected with 75% alcohol, and the mice were returned to their cages, covered with sterile dressings, kept warm until they woke up, and then continued to be housed for observation. Randomized dosing began when the average tumor volume reached about 150 mm³. At the end of the experiment, mice were intraperitoneally injected with 0.5% sodium pentobarbital solution at a dose of 120 mg/kg to euthanize them. Their tibia and tumor tissues were removed, fixed with 10% formaldehyde, paraffin sections were made, stained with HE, and the histological morphology and invasion of the tumors were observed under a microscope. The expression of proliferation indicators such as Ki-67 and PCNA in tumor cells was detected by immunohistochemistry. The experimental grouping and administering plan are reported in the table below.

Animal grouping and administering plan:

**Table 1: Pharmacodynamic test**

| Group | N¹ | Compound therapy | Dose (mg/kg) | Volume parameters in administrati on (µL/g)² | Routes of administr ation | Frequency of administrat ion³ |
|---|---|---|---|---|---|---|
| 1 | 6 | Control group | -- | 10 | PO | QD × 25 |
| 2 | 6 | Compound D | 1 | 10 | PO | QD × 25 |
| 3 | 6 | PD-1 antibody | 5 | 1 | I.P. | QW |
| 4 | 6 | Compound D + PD-1 antibody | 1mg/kg compound D, P.O., QD + 5mg/kg PD-1, I.P., QW | | | |

| | | | | | | |
|---|---|---|---|---|---|---|
| Note: 1. N: Number of mice per group. 2. Administering volume: 10 µL/g based on the weight of mice. If the weight loss exceeds 15%, stop administering immediately and resume it after the weight loss returns to less than 10%. 3. The experimental period may be adjusted according to the tumor volume. | | | | | | |

Animal housing: After the arrival of animals, they were housed in the experimental environment for 3-7 days before starting the experiment. The animals were housed in independent ventilation cages (IVCs) (3 per cage) in SPF animal rooms. All cages, bedding, and drinking water were sterilized before use. All laboratory personnel wore protective clothing and latex gloves when operating in the animal room. The animal information card for each cage indicated the number of animals in the cage, gender, strain, receipt date, administering plan, experiment number, group, and start date of the experiment. Cages, feed, and drinking water were replaced twice a week. The housing environment and lighting conditions were as follows:
Temperature: 20~26°C
Humidity: 40~70%
Lighting cycle: 12 hours of light, 12 hours of no light
Feed composition: The feed met the identification standards for laboratory animal food. The maximum content of pollutants was within a controllable range and the manufacturer was responsible for routine inspection. Drinking water was autoclaved.
Animal grouping: Animals were weighed before administration and tumor volumes were measured. Randomized grouping was based on tumor volumes (randomized block design).
Observation: The formulation and any modification of this experimental plan was implemented only after evaluation and approval by the Shanghai WuXi AppTec Institutional Animal Care and Use Committee (IACUC). The use and welfare of laboratory animals were carried out in accordance with the rules of the Association for Assessment and Accreditation of Laboratory Animal Care (AAALAC). The health status and mortality of animals were monitored daily. Routine examinations included observing the effects of tumor growth and drug treatment on their daily behavioral manifestations, such as behavioral activities, food and water intake, weight changes (weight measured twice a week), appearance signs or other abnormalities. The number of animal deaths and side effects within the group were recorded based on the number of animals in each group.
Experimental indicators: were used to examine whether tumor growth was inhibited, delayed or cured. The tumor diameter was measured twice a week with a vernier caliper. The calculation equation for tumor volumes is: V = 0.5a × b², where a and b represent the major and minor diameters of the tumor, respectively.

The antitumor efficacy of the compound was evaluated by TGI (%) or tumor proliferation rate T/C (%). TGI (%) reflects the tumor growth inhibition. Calculation of TGI (%): TGI (%) = [1 - (average tumor volume at the end of administering in a treatment group - average tumor volume at the start of administering in this treatment group)/(average tumor volume at the end of treatment in the solvent control group - average tumor volume at the start of treatment in the solvent control group)] × 100%.

Tumor proliferation rate T/C (%): The calculation equation is as follows: T/C (%) = Tᵢ/Vᵢ × 100%. Where Vᵢ is the average tumor volume of the solvent control group at a certain measurement, and Tᵢ is the average tumor volume of the treatment group at the same measurement.

Experiment termination: When the animal's health condition continued to deteriorate, or the tumor volume exceeded 3,000 mm³, or there was a serious disease, or pain, the animal was euthanized. In the following cases, the veterinarian was notified to give euthanasia:
Significant emaciation, with a weight loss of more than 20%;
Lack of free access to food and water;
The animals showed the following clinical manifestations and continued to deteriorate:
   Piloerection
   Hunched back
   White ears, nose, eyes or feet
   Shortness of breath
   Twitch
   Continuous diarrhea
   Dehydration
   Slow action
   Phonation

Data analysis: All data were expressed as Mean + SEM and statistically analyzed by one-way ANOVA using the statistical analysis software Graphpad10. *P < 0.05 was considered a statistically significant difference. The test results are reported in Table 2.

**Table 2: Statistics of tumor volume data**

| Group | Tumor volume (mm³) | | | | | | | | |
|---|---|---|---|---|---|---|---|---|---|
| | Day | 0 | 4 | 7 | 10 | 14 | 18 | 21 | 25 |
| 1 | Mean | 21.1 | 35.9 | 45.7 | 64.6 | 92.5 | 130.4 | 227.4 | 317.1 |
| | SEM | 1.07 | 2.96 | 4.53 | 8.05 | 13.4 | 27.1 | 47.2 | 64.7 |
| 2 | Mean | 21.6 | 35.7 | 41.3 | 52.2 | 68.7 | 85.2 | 136.9 | 225.6 |
| | SEM | 0.76 | 2.21 | 2.73 | 4.39 | 8.16 | 10.8 | 17.2 | 24.9 |
| 3 | Mean | 19.3 | 30.1 | 36.0 | 43.1 | 66.2 | 69.9 | 155.3 | 201.1 |
| | SEM | 0.53 | 1.63 | 3.40 | 2.45 | 7.60 | 6.90 | 15.0 | 19.4 |
| 4 | Mean | 22.2 | 24.4 | 27.1 | 29.2 | 34.5 | 39.7 | 46.3 | 80.5 |
| | SEM | 0.35 | 2.32 | 2.07 | 1.38 | 1.30 | 2.97 | 2.98 | 4.72 |

In the 25-day *in vivo* efficacy study using the tibial orthotopic syngeneic tumor model of DUNN cells in C3H mice, tumor-volume changes during dosing for each group are shown in Table 2 and Figure 1. The tumor growth inhibition (TGI) was 30.9% for compound D monotherapy (1 mg/kg, oral, once daily) , 38.6% for anti-PD-1 antibody monotherapy (5 mg/kg, intraperitoneal, once daily ;*P<0.05 vs. control), and 80.4% for the combination of compound D (1 mg/kg, oral, once daily) with the anti-PD-1 antibody (5 mg/kg, intraperitoneal, once daily; ***P<0.001 vs. control).

The results show that, relative to the control group, compound D monotherapy did not reach statistical significance; anti-PD-1 monotherapy achieved statistical significance ; and the combination of compound D with anti-PD-1 antibody was statistically significant. Compared with either monotherapy, the combination exhibited markedly enhanced antitumor activity.

Although preferred examples have been described hereinabove, it will be apparent to those skilled in the art that modifications may be made without departing from the present disclosure. Such modifications are considered to be possible variants encompassed within the scope of the present disclosure.

## Claims

1. A pharmaceutical composition comprising a PRMT5 inhibitor of Formula (I) or Formula (II) as a first active ingredient and a PD-1/PD-L1 inhibitor as a second active ingredient: 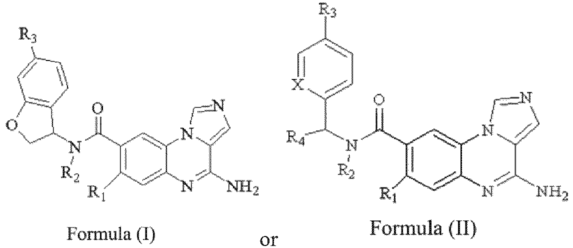 wherein, in Formula (I) or Formula (II),
R₁ is H, halogen, C₁-C₆ alkyl, C₁-C₆ haloalkyl, or CN;
R₂ is H, C₁-C₆ alkyl, C₁-C₆ haloalkyl, C₁-C₆ deuteroalkyl, or C₃-C₆ cycloalkyl; and
R₃ is H, halogen, C₁-C₆ alkyl, C₁-C₆ haloalkyl, C₁-C₆ haloalkoxy, or SF₅;
in Formula II, R₄ is hydrogen or C₁-C₆ alkyl; X is CR₅ or N; and
wherein, R₅ is hydrogen, halogen, C₁-C₆ alkyl, C₁-C₆ haloalkyl, hydroxyl, -NH₂, or CN.

2. The pharmaceutical composition according to claim 1, wherein, in Formula (I) or Formula (II):
R₁ is hydrogen, halogen, C₁-C₆ alkyl, or C₁-C₆ haloalkyl;
R₂ is hydrogen, C₁-C₆ alkyl, C₁-C₆ haloalkyl, C₁-C₆ deuteroalkyl, or C₃-C₆ cycloalkyl; and
R₃ is hydrogen, halogen, C₁-C₆ haloalkyl, C₁-C₆ haloalkoxy, or SF₅;
R₄ is hydrogen or methyl; and
in Formula II, X is CH or N.

3. The pharmaceutical composition according to claim 1, wherein, R₁ is hydrogen or fluorine.

4. The pharmaceutical composition according to claim 1, wherein, R₂ is cyclopropyl, methyl or deuterated methyl.

5. The pharmaceutical composition according to claim 1, wherein, R₃ is CF₃.

6. The pharmaceutical composition according to claim 1, wherein, R₄ is hydrogen or methyl.

7. The pharmaceutical composition according to claim 1, wherein, X is N.

8. The pharmaceutical composition according to claim 1, wherein, the PRMT5 inhibitor as the first active ingredient is selected from the group consisting of the following compounds and combinations thereof:

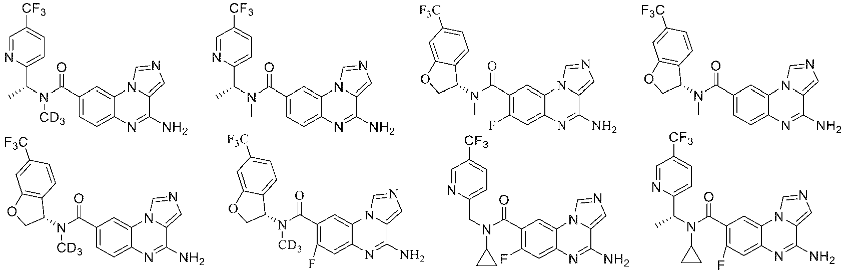

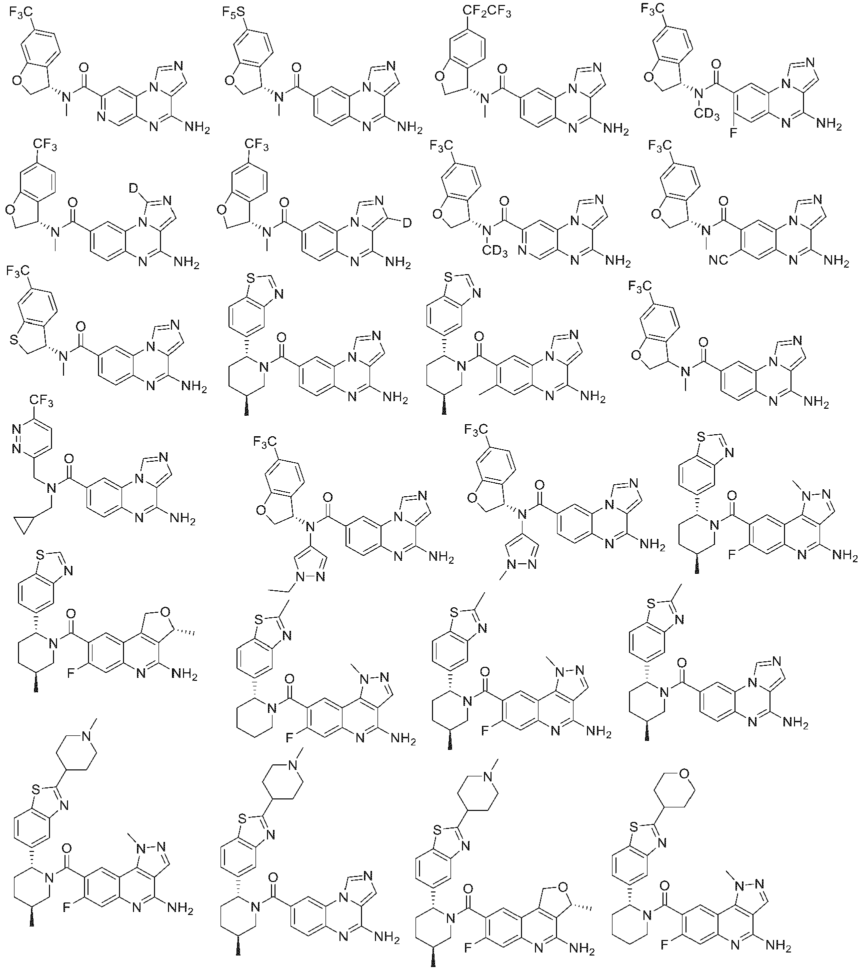

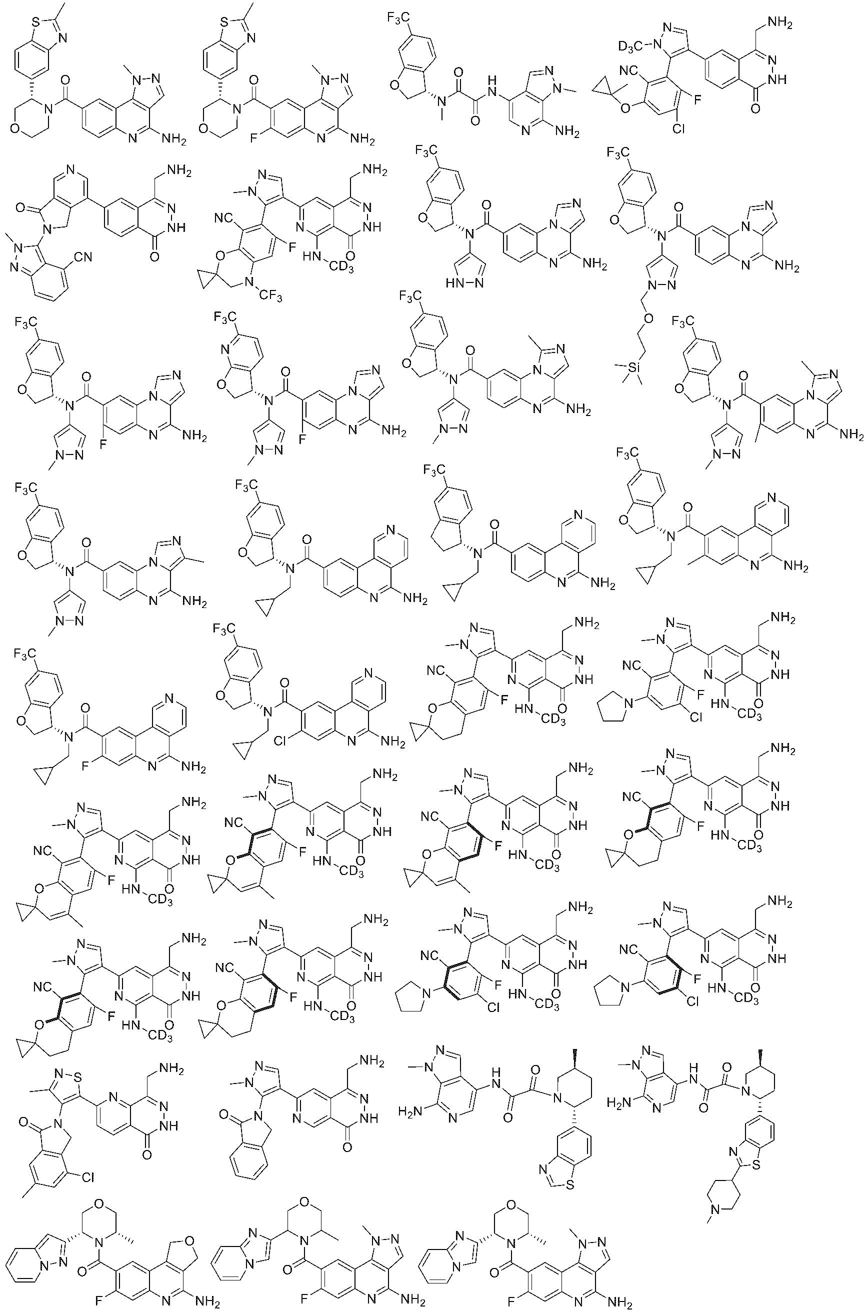

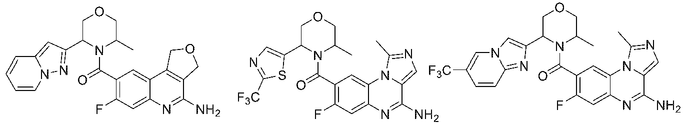

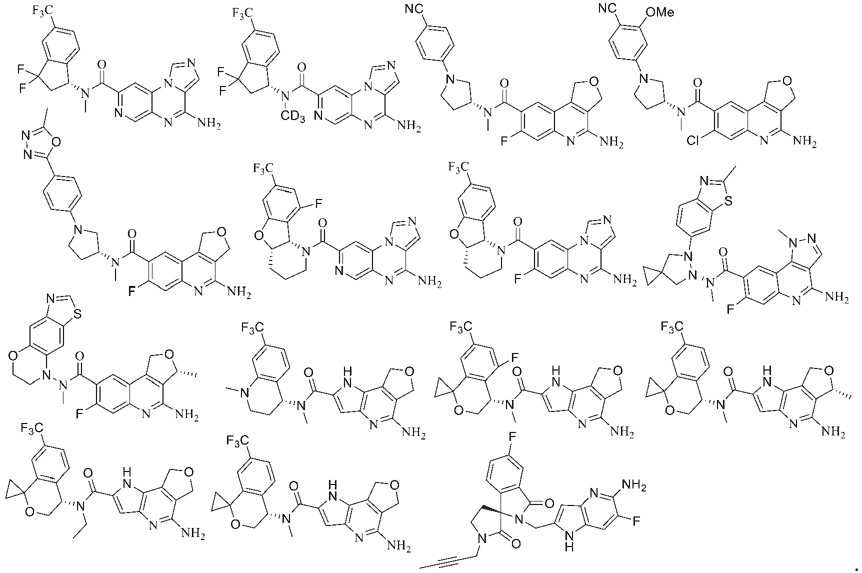

9. The pharmaceutical composition according to any one of claims 1 to 8, wherein, the PD-1/PD-L1 inhibitor as the second active ingredient is a small molecule compound, nucleic acid, peptide, protein, antibody, peptide antibody, double antibody, mini antibody, single chain fragment variable (ScFv) or a fragment or variant or any combination thereof that can be used to interfere with the interaction between PD-1 and PD-L1 to stimulate antitumor immune response, but is not limited by any binding theory.

10. The pharmaceutical composition according to claim 9, wherein, the PD-1/PD-L1 inhibitor as the second active ingredient is an antibody selected from anti-PD-1 antibody (or "PD-1 antibody" for short) and anti-PD-L1 antibody (or "PD-L1 antibody" for short).

11. The pharmaceutical composition according to any one of claims 9-10, wherein, the anti-PD-1/PD-L1 inhibitor is a monoclonal PD-1 antibody selected from nivolumab (OPDIVO^{®}), Pembrolizumab (Keytruda^{®}), Pidilizumab, Tislelizumab (Bai Ze'an^{®}), Sintilimab (Tyvyt^{®}), Penpulimab (Anniko^{®}), Dostarlimab (Jemperli^{®}), Cemiplimab (Libtayo^{®}), Toripalimab (Loqtorzi^{®}), Tislelizumab (Tevimbra^{®}), Retifanlimab (Zynyz^{®}), Spartalizumab (PDR001), Camrelizumab (SHR-1210), MEDI0680 or RMP1-14 (rat IgG2A) or any combination thereof.

12. The pharmaceutical composition according to claim 9, wherein, the PD-1/PD-L1 inhibitor is a monoclonal PD-L1 antibody selected from Atezolizumab (TECENTRIQ^{®}), Envafolimab (Envida^{®}, KN035), Avelumab (BAVENCIO^{®}), Durvalumab (Imfinzi^{®}) and BMS936559 or any combination thereof.

13. The pharmaceutical composition according to claim 9, wherein, the anti-PD-1/PD-L1 inhibitor is a small molecule compound selected from ASC61, BPI-371153, AN4005, INCB086550, MAX-10181 or Emdiphene (IMMH-010) or any combination thereof.

14. A method for treating cancers or tumors, comprising administering the pharmaceutical composition according to any one of claims 1 to 13 to a subject in need thereof.

15. The method according to claim 14, wherein, the tumor or cancer is selected from: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, bile duct cancer, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, epidermal squamous cell carcinoma, nasopharyngeal carcinoma, neuroblastoma, endometrial cancer, head and neck cancer, and cervical cancer.

16. The method according to claim 14, wherein, the cancer is metastatic cancer.

17. The method according to claim 15, wherein, the metastatic cancer is brain metastatic cancer.
